Europäisches Patentamt

European Patent Office

Office européen des brevets

(11) Veröffentlichungsnummer: **0 304 007**
**A1**

## (12) EUROPÄISCHE PATENTANMELDUNG

(21) Anmeldenummer: 88113256.7

(22) Anmeldetag: 16.08.88

(51) Int. Cl.4: **H05B 3/10 , H05B 3/56 ,
H05B 3/18**

(30) Priorität: 20.08.87 DE 3727732

(43) Veröffentlichungstag der Anmeldung:
**22.02.89 Patentblatt 89/08**

(84) Benannte Vertragsstaaten:
**AT BE CH DE ES FR GB GR IT LI LU NL SE**

(71) Anmelder: **Asea Brown Boveri
Aktiengesellschaft
Kallstadter Strasse 1
D-6800 Mannheim-Käfertal(DE)**

(72) Erfinder: **Falk, Winfried
Eduard-von-Heuss-Strasse 22
D-6501 Bodenheim(DE)**

(74) Vertreter: **Rupprecht, Klaus, Dipl.-Ing. et al
c/o BBC Brown Boveri Aktiengesellschaft
ZPT Postfach 100351 Kallstadter Strasse 1
D-6800 Mannheim 1(DE)**

(54) Einrichtung zur elektrischen Beheizung von Rohren, Behältern und dergleichen.

(57) Jede Heizleitung (10) besitzt zwei im Abstand zueinander angeordnete Heizleiter (11, 13) zwischen denen ein weiterer Leiter (Kaltleiter 12) angeordnet ist, dessen Querschnitt so bemessen ist, daß die Temperaturerhöhung bei Stromdurchfluß gegenüber der Temperaturerhöhung der Heizleiter (11, 13) vernachlässigbar ist. Die drei Leiter liegen im wesentlichen in einer Ebene und können dabei einzeln isoliert sein (Einzelisolierung 14) oder in einer gemeinsamen Isolierung untergebracht sein. Durch die erfindungsgemäße Ausgestaltung besteht die Möglichkeit die drei Leiter in geeigneter Weise miteinander zusammenzuschalten, wodurch mit einer Leitung durch unterschiedliche Zusammenschaltungen vier Widerstandswerte erreicht werden können.

Fig. 1

## Einrichtung zur elektrischen Beheizung von Rohren, Behältern und dergleichen

Die Erfindung betrifft eine Einrichtung zur elektrischen Beheizung von Rohren, Behälter und dgl., mit einer Heizleitung, die wenigstens einen Heizleiter aufweist.

Bekannte Heizleitungen sind einadrig aufgebaut. Sie besitzen einen Widerstandsleiter, der von einer inneren Isolierung meist aus Polytetrafluoräthylen umgeben ist, welches selbst wiederum in ein Schutzgeflecht aus elektrisch leitendem Material, meist aus vernickeltem Kupfer eingefügt ist. Darüberhinaus ist um dieses Schutzgeflecht eine äußere Isolierung aus Polytetrafluoräthylen vorgesehen, die von einem Montageschutz aus isolierendem Kunststoff, vorzugsweise aus Polyamid umgeben ist. Heizleitungen oder Heizkabel, die im Ex- oder auch im Nicht-Ex-Bereich verwendet werden, besitzen einen Heizleiter, der von einer Mineralisolierung, vorzugsweise aus gepreßtem Magnesiumoxydpulver umgeben ist, auf dessen Außenseite ein Metallmantel und ggf. auch ein zusätzlicher Kunststoffmantel angebracht ist.

Alle diese bekannten Heizleiter besitzen nur einen Widerstandsleiter im Inneren des Heizleiters, so daß für unterschiedliche Anwendungszwecke auch Leiter mit unterschiedlichem Querschnitt und Widerstandswerten vorgesehen und vorgeschalten werden müssen. Dadurch sind die Lagerhaltungskosten relativ hoch.

Aufgabe der Erfindung ist es, eine Einrichtung der eingangs genannten Art zu schaffen, bei der die Lagerhaltungskosten reduziert und der Montageaufwand ebenfalls verringert ist.

Diese Aufgabe wird erfindungsgemäß dadurch gelöst, daß die Heizleitung wenigstens zwei Heizleiter und wenigstens einen weiteren Leiter aufweist, von denen je ein weiterer Leiter je zwei Heizleitern zugeordnet ist.

Die beiden Heizleiter besitzen den für die Beheizung und für die Erzielung einer ausreichenden Leistung (Temperatur) erforderlichen Widerstandswert, wogegen der weitere Leiter ein sog. Kaltleiter ist, also ein Leiter, der sich dann, wenn er im Betrieb von Strom durchflossen ist nicht oder nur geringfügig erwärmt.

Jeder Heizleiter und jeder weitere Leiter können dabei von einer Einzelisolierung umgeben sein, wobei um die einzeln isolierten Leiter eine gemeinsame Leiterisolation, das Schutzgeflecht und der sog. Außenmantel herumgelegt sind. Es besteht auch die Möglichkeit, anstatt der Einzelisolierung auch eine gemeinsame Leiterisolation gemäß kennzeichnendem Teil des Anspruches 3 vorzusehen, wodurch eine vereinfachte Herstellung bewirkt wird. Die Einzelisolierung und die gemeinsame Isolierung ist aus Material wie Polytetrafluoräthylen gebildet.

Eine bevorzugte Ausgestaltung der Erfindung geht dahin, daß innerhalb einer Heizleitung zwei Heizleiter und dazwischen der Kaltleiter vorgesehen sind.

Erfindungsgemäß also befinden sich in einer einzigen Heizleitung mehrere Leiter, die dann auch in geeigneter Weise miteinander zusammengeschaltet werden können, um bei einer einzigen Heizleitung unterschiedliche Widerstandswerte zu erzielen.

Anhand der Zeichnung, in der zwei Ausführungsbeispiele der Erfindung dargestellt sind, sollen die Erfindung sowie weitere vorteilhafte Ausgestaltungen und Verbesserungen und weitere Vorteile näher erläutert und beschrieben werden.

Es zeigt:

Figur 1 eine Seitenansicht einer erfindungsgemäßen Heizleitung gemäß einem ersten Ausführungsbeispiel

Figur 2 eine Heizleitung nach einem zweiten Ausführungsbeispiel

Figur 3 eine Schnittansicht durch einen Heizleiter gemäß Figur 1

Figur 4 einige Schaltungsbeispiele zum Zusammenschalten einer Heizleitung gemäß der Erfindung und

Figur 5 eine Schnittsicht durch ein Rohr mit vier aufgebrachten Heizleitungen.

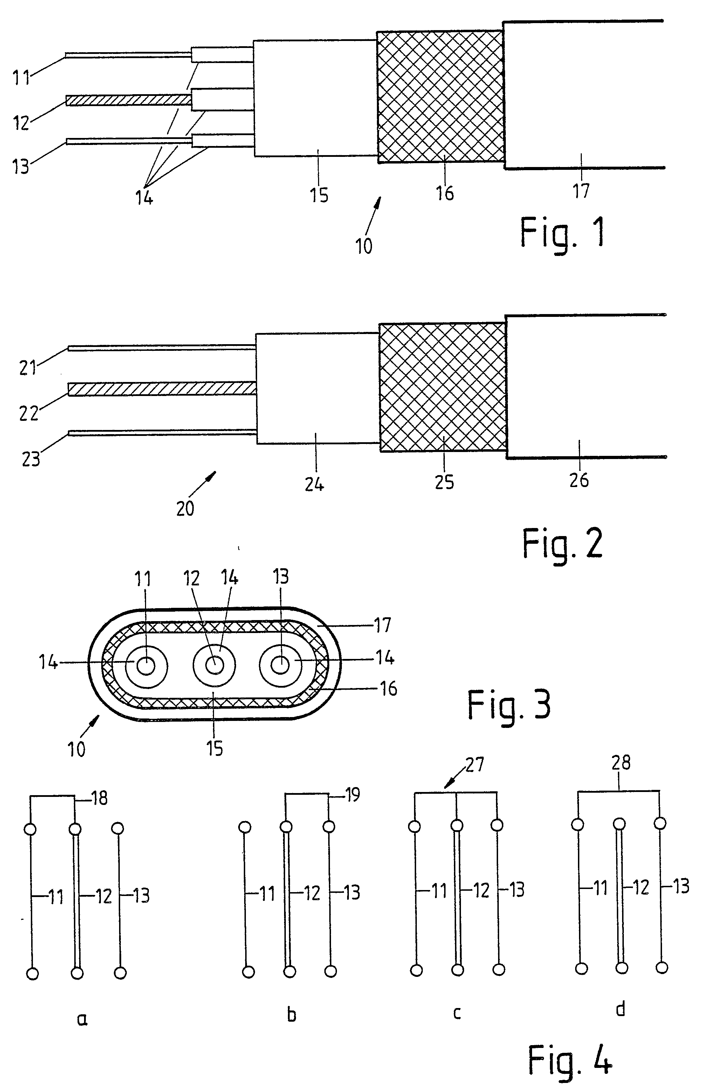

Die erfindungsgemäße Heizleitung gemäß Figur 1 besitzt einen ersten Heizleiter 11, einen sog. Kaltleiter 12 und einen zweiten Heizleiter 13, wobei die beiden Heizleiter 11 und 13 in Abstand zueinander in einer Ebene liegend angeordnet sind; dazwischen befindet sich der Kaltleiter 12. Der Kaltleiter ist deshalb als Kaltleiter bezeichnet, weil sein Querschnitt so bemessen ist, daß er im Gegensatz zu den Heizleitern 11 und 13 keine wesentliche Temperaturerhöhung erfährt, wenn Strom durch ihn hindurchfließt.

Jeder der drei Leiter, die beiden Heizleiter 11 und 13 und der Kaltleiter 12, besitzt eine eigene Leiterisolation 14, die von einer gemeinsamen Leiterisolation 15 umgeben ist, in der die einzelnen Leiter 11 bis 13 mit ihren Leiterisolationen 14 eingebettet sind. Um die gemeinsame Leiterisolation 15 herum ist ein Schutzgeflecht 16 aus elektrisch leitendem Material, vorzugsweise aus vernickeltem Kupfer, herum angeordnet und um den Schutzmantel 16 herum ist ein Außenmantel 17 gelegt. Die Leiterisolation 14 besteht ebenso wie die gemeinsame Isolation 15 vorzugsweise aus einem Fluorkunststoff, wie Polytetrafluoräthylen, Kapton oder FEP. Ebenfalls besteht der Außenmantel aus einem Fluorkunststoffmaterial.

Die Ausgestaltung gemäß Figur 2 zeigt drei Leiter, einen ersten Heizleiter 21, einen zweiten Heizleiter 23 und dazwischen befindlich einen Kaltleiter 22, welche drei Leiter den Leitern 11, 12, und 13 entsprechen. Anstatt einer Einzelisolierung 15 ist um die Leiter 21 bis 23 eine gemeinsame Leiterisolation 24 herum angeordnet, so daß die drei Leiter 21 bis 23 in dieser gemeinsamen Leiterisolation 24 eingebettet sind. Um diese gemeinsame Leiterisolation herum befindet sich wieder ein Schutzgeflecht 25, das dem Schutzgeflecht 16 entspricht, und die gesamte Heizleitung 20 besitzt an ihrer Außenfläche bzw. an ihrem Außenumfang einen Außenmantel 26. Die Leiterisolation 24 und der Außenmantel 26 bestehen aus dem gleichen Material wie oben erwähnt: Fluorkunststoffe, insbesondere Polytetrafluoräthylen.

Aus der Figur 3 ist der Querschnitt der Heizleitung 10 der Figur 1 ersichtlich. Man erkennt den Heizleiter 11, den Kaltleiter 12 und den zweiten Heizleiter 13, welche drei Leiter mit je einer Einzelisolation 14 aus Polytetrafluoräthylen versehen sind. Diese drei Leiter sind in einer gemeinsamen Isolierung 15 eingebettet, die vom Schutzmantel 16 und zusätzlich vom Außenmantel 17 umgeben ist.

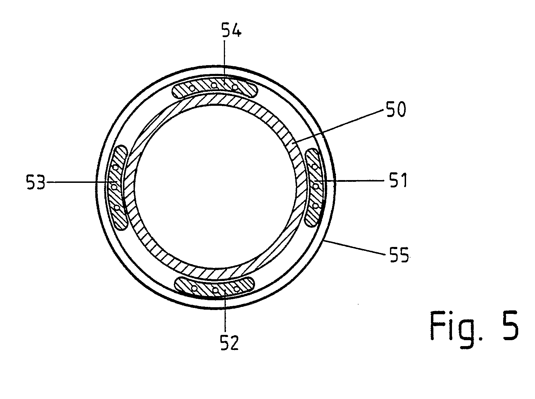

Die Figur 5 zeigt die mechanische Zuordnung mehrerer Heizleitungen zu einem Rohr 50. Auf der Außenfläche dieses Rohres 50 sind vier Heizleitungen 51, 52, 53 und 54 herum in gleichem Abstand zueinander bzw. gleichmäßig am Umfang verteilt angeordnet, wobei alle vier Heizleitungen entweder nach Figur 1 oder Figur 2 aufgebaut sind. Um die Heizleiter 51 bis 54 ist ein Glasseidenband 55 herum geschlungen, welches die vier Heizleiter gegen die Außenfläche des Rohres preßt.

Die Figur 4 zeigt vier Schaltungsanordnungen, wie die einzelnen Leiter innerhalb einer Heizleitung zusammengeschaltet werden können.

Nach Figur 4 a können der Heizleiter 11 und der Kaltleiter 12 über die Leitungsverbindung 18 verbunden sein; gemäß Figur 4 b ist eine Verbindung möglich zwischen dem Kaltleiter 12 und dem Heizleiter 13 über eine Verbindungsleitung 19; gemäß Figur 4 c sind mit einer Verbindungsleitung 27 alle drei Leiter 11, 12 und 13 miteinander verbunden, und gemäß Figur 4 d erhält man eine Verbindung lediglich der Heizleiter 11 und 13 mittels der Verbindungsleitung 28.

Wenn die beiden Heizleiter 11 und 13 gleich ausgebildet sind, dann wird sich der Widerstandswert zwischen den beiden Ausführungen bzw. Schaltungen nach Figur 4 a und 4 b nicht unterscheiden.

Es besteht jedoch die Möglichkeit, daß zur Erzielung einer erhöhten Vielfalt der Heizleiter 11 einen ersten Widerstandswert und der Heizleiter 13 einen zweiten Widerstandswert aufweisen und demgemäß erhält man je nach Schaltung gemäß Figur 4 a bzw. Figur 4 b unterschiedliche Gesamtwiderstandswerte. In gleicher Weise erhält man ebenfalls unterschiedliche Gesamtwiderstandsbeiwerte, wenn gemäß Figur 4 c alle Leiter parallel geschaltet und gemäß Figur 4 d die Heizleiter in Serie geschaltet werden.

Wenn z.B. der Leiterdurchmesser des Heizleiters 11 1,2 mm (entspricht 0,11 Ohm/m bei einer siebenadrigen Litze) und der Durchmesser des Heizleiters 13 0,9 mm (entspricht 0,25 Ohm/m bei einer siebenadrigen Litze) beträgt, wobei der Kaltleiter eine verzinnte Kupferlitze mit 2.5 mm$^2$ Querschnitt ist, dann erhält man bei der Zusammenschaltung nach der Figur 4 a einen Widerstand von 0,11 Ohm/m, bei der Figur 4 b 0,25 Ohm/m, bei der Parallelschaltung Figur 4 c 0,076 Ohm/m und bei der Serienschaltung Figur 4 d 0,36 Ohm/m. Damit kann man mit einer einzigen Heizleitung vier Widerstandswerte erzielen, was eine erhebliche Einsparung an Heizleitern darstellt. Wenn man z.B. fünf Heizleitungen mit unterschiedlichen Heizleiterquerschnitten und dadurch bestimmten Widerstandswerten auf Lager hält, kann man durch geeignete Zusammenschaltung gemäß Figur 4 a bis 4 d insgesamt 20 Widerstandswerte, gemessen in Ohm/m, erhalten. Die Widerstandswerte bedingen dann eine bestimmte Heizleistung bzw. Warmeabgabe.

Durch mehr Auswahl bei den Widerstandswerten entfällt der Einsatz von evtl. benötigten Transformatoren, Energiestellern usw., im Vergleich zu solchen Heizleitungen, bei denen lediglich ein Heizleiter für sich isoliert innerhalb der Heizleitung vorgesehen ist. Darüberhinaus ist die Montage erleichtert und zwar weil in vielen Fällen eine Einfachverlegung möglich ist. So kann z.B. auch eine Schlaufenverlegung durch die Ausgestaltungen und Schaltungen gemäß Figur 4 a bis 4 d vermieden werden.

## Ansprüche

1. Einrichtung zur elektrischen Beheizung von Rohren, Behältern und dgl., mit mindestens einer Heizleitung mit darin eingebetteten Heizleitern, <u>dadurch gekennzeichnet</u>, daß die Heizleitung wenigstens zwei Heizleiter (11, 13; 21, 23) und wenigstens einen weiteren Leiter (Kaltleiter 12, 22) aufweist, von denen je ein weiterer Leiter (Kaltleiter) je zwei Heizleitern zugeordnet ist, und daß die einzelnen Leiter nebeneinander in einer Ebene liegend angeordnet sind.

2. Einrichtung nach Anspruch 1, dadurch gekennzeichnet, daß jeweils ein weiterer Leiter (Kaltleiter 12) zwischen zwei Heizleitern (11 und 13) angeordnet ist.

3. Einrichtung nach Anspruch 2, dadurch gekennzeichnet, daß der Querschnitt jedes weiteren Leiters (Kaltleiter 12, 22) so bemessen ist, daß sein Temperaturanstieg im Vergleich zum Temperaturanstieg in den Heizleitern bei einem Stromdurchfluß vernachlässigbar ist.

4. Einrichtung nach einem der vorigen Ansprüche, dadurch gekennzeichnet, daß die beiden Heizleiter unterschiedliche Widerstandswerte aufweisen.

5. Einrichtung nach einem der vorigen Ansprüche, dadurch gekennzeichnet, daß jeder Leiter, Heizleiter (11, 13) und weiterer Leiter (Kaltleiter 12), mit einer Einzelisolierung (14) umgeben sind und daß alle Leiter mit ihren Einzelisolierungen innerhalb einer gemeinsamen Isolierung (15), einem Schutzgeflecht (16) und einem Außenmantel (17) eingebettet sind.

6. Einrichtung nach einem der Ansprüche 1 bis 4, dadurch gekennzeichnet, daß die Heizleiter und der weitere Leiter (Kaltleiter) in einer gemeinsamen Isolierung (24) eingebettet sind, die von einem Schutzgeflecht (25) und einem Außenmantel (26) umgeben ist.

7. Einrichtung nach einem der vorigen Ansprüche, dadurch gekennzeichnet, daß die Isolierungen, nämlich die Einzelisolierung (14), die gemeinsame Isolierung (15, 24) und der Außenmantel (17, 26), aus Fluorkunststoffen, vorzugsweise Polytetrafluoräthylen, bestehen.

Fig. 1

Fig. 2

Fig. 3

Fig. 4

Fig. 5

| | **EINSCHLÄGIGE DOKUMENTE** | | EP 88113256.7 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A - 1 690 578 (KABEL- UND METALLWERKE)<br>  * Seite 5, Zeilen 16-26; Fig. 2,3 *<br>-- | 1,2,5,6 | H 05 B 3/10<br>H 05 B 3/56<br>H 05 B 3/18 |
| A | US - A - 4 435 639 (GUREVICH)<br>  * Spalte 2, Zeilen 41-52; Fig. 1,2 *<br>-- | 1,2,5,6 | |
| A | EP - A2/A3 - 0 008 235 (EATON)<br>  * Seite 12, Zeilen 6-27; Fig. 4 *<br>-- | 1,2,5,6 | |
| A | US - A - 4 659 913 (MIDGLEY)<br>  * Zusammenfassung; Fig. 6,22-28 *<br>-- | 1,2,5,6 | **RECHERCHIERTE SACHGEBIETE (Int. Cl.4)** |
| A | DE - A1 - 3 243 061 (HEW)<br>  * Zusammenfassung; Fig. 1 *<br>---- | 1,2,5-7 | H 05 B 1/00<br>H 05 B 3/00 |

Der vorliegende Recherchenbericht wurde für alle Patentansprüche erstellt.

| Recherchenort | Abschlußdatum der Recherche | Prüfer |
|---|---|---|
| WIEN | 31-10-1988 | TSILIDIS |